# EUROPEAN PATENT SPECIFICATION

(11) **EP 1 174 411 B1**
(45) Date of publication and mention of the grant of the patent: **08.10.2003**
(21) Application number: 01116794.7
(22) Date of filing: 23.07.2001
(51) Int. Cl.: C07C 51/235, C07C 65/40

(54) **Process for the preparation of aromatic carboxylic acid**
Verfahren zur Herstellung von aromatischen Karbonsäure
Procédé de fabrication d'acides carboniques aromatiques

(30) Priority: 21.07.2000 JP 2000220964
(43) Date of publication of application: 23.01.2002
(73) Proprietor: Fuji Photo Film Co., Ltd., Kanagawa-ken, 250-0123 (JP)
(72) Inventor: Nishikawa, Naoyuki, Minami-ashigara-shi, Kanagawa 250-0123 (JP)
(74) Representative: HOFFMANN - EITLE

(56) References cited:
- DE-A- 19 716 822
- US-A- 5 087 672
- PATENT ABSTRACTS OF JAPAN vol. 008, no. 172 (C-237), 9 August 1984 (1984-08-09) & JP 59 070643 A (TEIKOKU KAGAKU SANGYO KK), 21 April 1984 (1984-04-21)

## Description

### FIELD OF THE INVENTION

The present invention relates to a process for the preparation of an aromatic carboxylic acid, which can be advantageously used as an intermediate of a polymerizable liquid crystal compound or as a dental additive.

### BACKGROUND OF THE INVENTION

A polymer liquid crystal has been synthesized by a polymerization reaction of a polymerizable liquid crystal compound. The polymerizable liquid crystal compound is described in Makromol. Chem., 179(1978), 273; and *ibid.* 183(1982), pp. 2,311.

A liquid crystal compound includes a rod-like liquid crystal compound and a discotic liquid crystal compound. The polymerizable liquid crystal compound also includes a polymerizable rod-like liquid crystal compound and a polymerizable discotic liquid crystal compound.

The polymerizable rod-like liquid crystal compound is described in U.S. patent No. 5,087,672. The polymerizable rod-like liquid crystal compound is generally represented by the following formula (VIII): 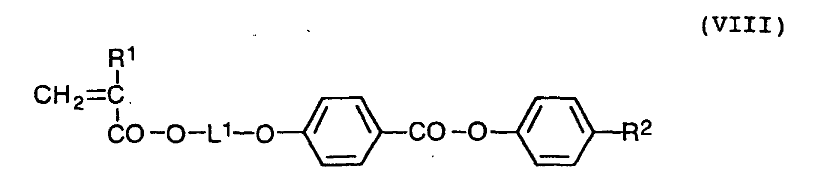 in which L¹ is an alkylene group; R¹ is hydrogen or methyl; and R² is a lower alkoxy group, cyano or a fluorine-substituted alkoxy group.

The polymerizable discotic liquid crystal compound has been used in the preparation of an optical compensatory sheet (particularly, for enlarging a viewing angle of TFT-LCD). The optical compensatory sheet using the polymerizable discotic liquid crystal compound is described in Optical Controllable Polymer of the Next Generation or Hyper-molecular Compound (written in Japanese) edited by Japan Polymer Society, 2000, pp. 41; and Bulletin of Japan Liquid Crystal Society (written in Japanese), vol. 1, pp. 45.

The polymerizable discotic liquid crystal compound is generally represented by the following formula (IX); 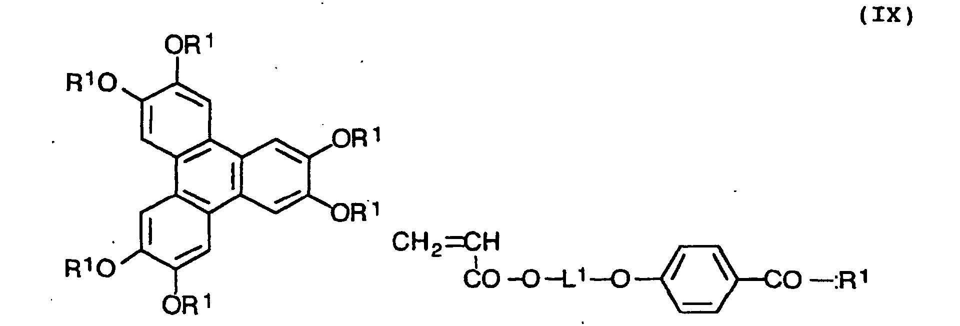 in which L¹ is an alkylene group.

In the preparation of the polymerizable liquid crystal compounds, an aromatic carboxylic acid represented by the following formula (X) can be used as an important intermediate: 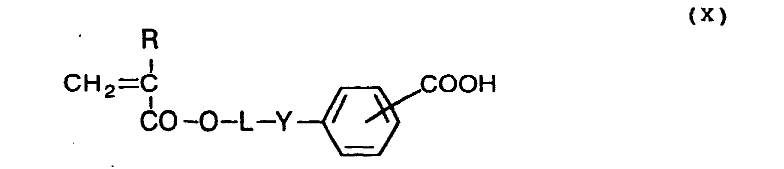 in which L is a divalent linking group having 2 to 20 carbon atoms; R is hydrogen or methyl; Y is oxygen or sulfur; and the benzene ring can have 1 to 3 substituent groups.

The aromatic carboxylic acid can also be used as an additive for a dental adhesive (described in Japanese Patent Provisional Publication No. 59(1984)-70643) to improve adhesion. The aromatic carboxylic acid for the dental adhesive is generally represented by the following formula (XI): 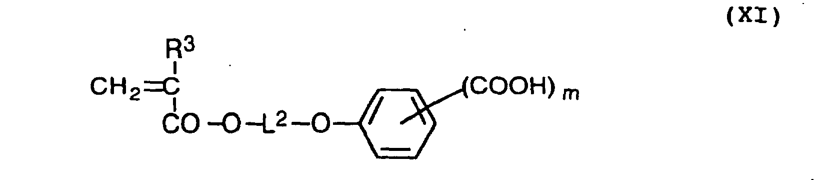 in which R³ is methyl or hydrogen atom; L² is an alkylene group having 2 to 4 carbon atoms; and m is 1, 2 or 3.

It is difficult to synthesize an aromatic carboxylic acid having a (meth)acryloyloxy group represented by the formula (X) or (XI) in a high yield.

A process for preparing the compound of the formula (V) in which Y is an oxygen atom is disclosed in Makromol. Chem., 179(1978), pp. 273; and *ibid.* 183(1982), pp. 2,311. The process comprises the steps of: reacting p-hydroxybenzoic acid with ω-halogenoalkyl-1-alcohol in the presence of sodium hydroxide (base) to prepare p-(ω)-hydroxyalkyloxy)benzoic acid, and condensing the prepared acid with acrylic acid under acidic condition to prepare the aimed compound. This process, however, has some defects. In the step for preparing p-(ω-hydroxyalkyloxy)benzoic acid, esterification proceeds together with etherification. Further, if some ω-halogenoalkyl-1-alcohol (e.g., 2-bromoethanol, 4-bromo-1-butanol) is used, a practical yield is not realized.

Another process is disclosed in Optical Controllable Polymer of the Next Generation or Hyper-molecular Compound (written in Japanese) edited by Japan Polymer Society, 2000, pp. 41; Japanese Patent Provisional Publication No. 7(1995)-306317; and Makromol. Chem., 190(1989), pp. 2, 255. The process comprises the steps of: reacting ethyl p-hydroxybenzoate with ω-halogenoalkyl alcohol to prepare ethyl p-(ω-hydroxyalkyloxy)benzoate, hydrolyzing the prepared ester with alkali to prepare p-(ω-hydroxyalkyloxy)benzoic acid, and then reacting the prepared acid with acryloyl chloride or methacryloyl chloride. This process is also unfavorable for practical preparation. Acryloyl chloride or methacryloyl chloride, which is used in the last step, is too lachrymal to use practically. Further, through the process, a hydrochloride adduct is produced as a by-product. Furthermore, like the above-described process, a practical yield is not realized if some ω-halogenoalkyl alcohol is used.

Japanese Patent Provisional Publication No. 59(1984)-70643 discloses a process comprising the steps of: reacting the compound represented by the following formula (XII) with acryloyl halide or methacryloyl halide in the presence of base, or otherwise reacting the compound represented by the following formula (XIII) with acrylic or methacrylic salt of alkali metal, to prepare the compound represented by the following formula (XIV); and then oxidizing the prepared compound to prepare the compound represented by the above-described formula (XI): 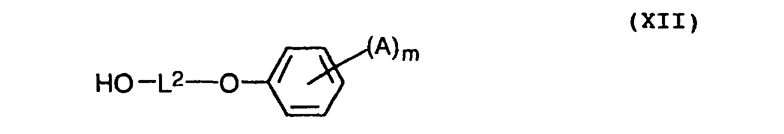 in which L² is an alkylene group having 2 to 4 carbon atoms; m is an integer of 1, 2 or 3; and A is methyl or formyl; 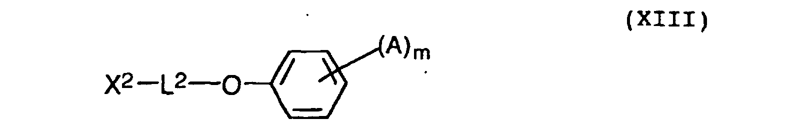 in which each of L², m and A is the same as in the formula (XII); and X² is a halogen atom; 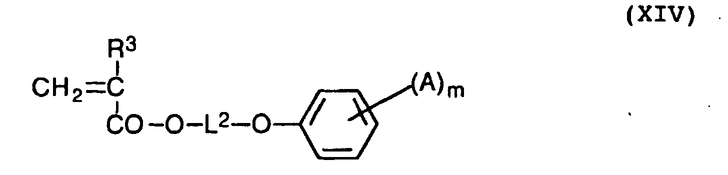 in which each of L², m and A is the same as in the formula (XII); and R³ is methyl or hydrogen.

Japanese Patent Provisional Publication No. 59(1984)-709643 discloses, as a reference example, a process for preparing the compound of the formula (XII) in which L² is ethylene or butylene, A is formyl, and m is 1. This process also has some problems. The process comprises many steps, and the product of each step is obtained from hydroxybenzaldehyde (starting material) in a low yield. Further, in the publication, Jones reagent and potassium permanganate are mentioned as examples of oxidizing agent, and actually Jones reagent is used in the working example. However, it is unfavorable to use Jones reagent in consideration of ecology. On the other hand, if potassium permanganate is used, a practical yield is hardly realized because the double bond of acrylic or methacrylic group is easily broken.

U.S. Patent No. 5,087,672 discloses a process for preparing the compound of the formula (V) in which Y is an oxygen atom. The process comprises the steps of: reacting p-hydroxybenzoic acid with ω-halogenoalkyl alcohol to prepare p-(ω-hydroxyalkyloxy)benzoic )benzoic acid, and condensing the prepared acid with acrylic acid to prepare the aimed compound. Further, in the patent, a process for preparing the compound of the formula (XI) is also disclosed. The process comprises the steps of: reacting p-hydroxybenzaldehyde with ω-halogenoalkyl alcohol to prepare p-(ω)-hydroxyalkyloxy)benzaldehyde, and condensing the prepared aldehyde with acrylic acid to prepare the aimed compound. In these disclosed processes, an oxidizing agent that does not affect polymerizable groups is used. As examples of such oxidizing agent, peracids (perbenzoic acid, peracetic acid, pertrifluoroacetic acid), permanganates, chromic acid, bromine, silver oxide and sodium chlorite are described. Among them, it is particularly preferred to use sodium chlorite in phosphate buffer solution. However, also in these processes, a practical yield is hardly realized if some ω-halogenoalkyl alcohol is used.

WO00/05189 discloses a process for the preparation of the compound represented by the formula (XI), which comprises a reaction of p-hydroxybenzaldehyde with acryloyloxyalkyl-1-chloride to obtain p-(acryloyloxylalkyloxy)benzaldehyde and the above-mentioned Jones oxidation. However, it is unfavorable to use Jones reagent in consideration of ecology, as is described above.

German Patent Application No. 19716822A1 discloses a process of reacting a (meth)acrylic ester with phosgene to obtain chloroformate and converting chloroformate to acryloyloxyalkyl-1-chloride by using dimethylformamide as a catalyst. However, phosgene is poisonous. Further, phosgene causes one equivalent of hydrochloric gas and carbon dioxide gas in a reaction system.

### SUMMARY OF THE INVENTION

It is difficult to synthesize an aromatic carboxylic acid having a (meth)acryloyloxy group in a high yield according to a conventional process.

Further the conventional process has a low efficiency, since the process comprises many steps of taking out intermediates of reaction vessels.

An object of the present invention is to prepare an aromatic carboxylic acid by an efficient process that can be widely applied.

Another object of the invention is to prepare an aromatic carboxylic acid having a linking group (L in the formula (V)), which is difficult to be synthesized by a conventional process.

A further object of the invention is to prepare an aromatic aldehyde by applying a process of the preparation of an aromatic carboxylic acid.

The present invention provides a process for the preparation of an aromatic carboxylic acid represented by the formula (V), which comprises the steps of: synthesizing a sulfonic ester represented by the formula (II) by a reaction of a (meth)acrylic ester represented by the formula (I) with a sulfonyl donor; synthesizing an aromatic aldehyde represented by the formula (IV) by a reaction of the sulfonic ester represented by the formula (II) with an aromatic aldehyde represented by the formula (III); and synthesizing the aromatic carboxylic acid represented by the formula (V) by an oxidation of the aromatic aldehyde represented by the formula (IV): 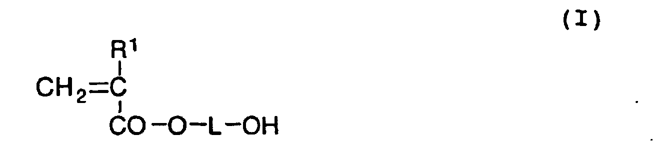 in which L is a divalent linking group having 2 to 20 carbon atoms selected from the group consisting of a divalent aliphatic group, a divalent aromatic group and a combination of two or more divalent aliphatic or aromatic groups combined with -O-, -S-, -CO-, -NH- or a combination thereof; and R¹ is hydrogen or methyl; 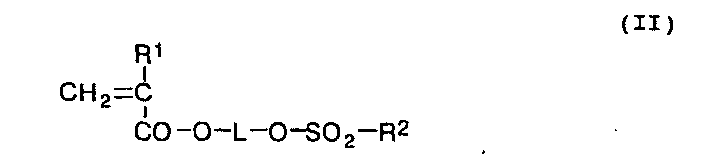 in which L is a divalent linking group having 2 to 20 carbon atoms selected from the group consisting of a divalent aliphatic group, a divalent aromatic group and a combination of two or more divalent aliphatic or aromatic groups combined with -O-, -S-, -CO-, -NH- or a combination thereof; R¹ is hydrogen or methyl; and R² is an aliphatic group or an aromatic group;

H-Y-Ar-CHO (III)

in which Y is oxygen or sulfur; and Ar is a divalent aromatic group; 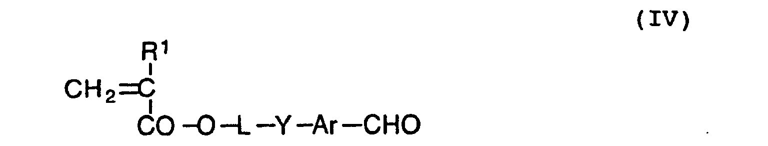 in which L is a divalent linking group having 2 to 20 carbon atoms selected from the group consisting of a divalent aliphatic group, a divalent aromatic group and a combination of two or more divalent aliphatic or aromatic groups combined with -O-, -S-, -CO-, -NH- or a combination thereof; R¹ is hydrogen or methyl; Y is oxygen or sulfur; and Ar is a divalent aromatic group; 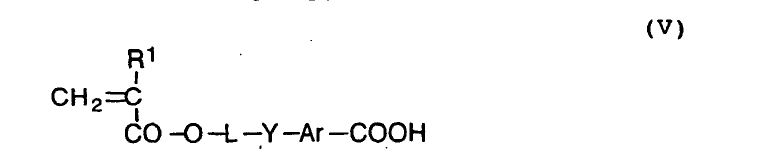 in which L is a divalent linking group having 2 to 20 carbon atoms selected from the group consisting of a divalent aliphatic group, a divalent aromatic group and a combination of two or more divalent aliphatic or aromatic,groups combined with -O-, -S-, -CO-, -NH- or a combination thereof; R¹ is hydrogen or methyl; Y is oxygen or sulfur; and Ar is a divalent aromatic group.

The invention also provides a process for the preparation of an aromatic aldehyde represented by the formula (IV), which comprises the steps of: synthesizing a sulfonic ester represented by the formula (II) by a reaction of a (meth)acrylic ester represented by the formula (I) with a sulfonyl donor; and synthesizing the aromatic aldehyde represented by the formula (IV) by a reaction of the sulfonic ester represented by the formula (II) with an aromatic aldehyde represented by the formula (III).

### DETAILED DESCRIPTION OF THE INVENTION

A (meth)acrylic ester represented by the formula (I) is used as the starting material at the first step of the process.

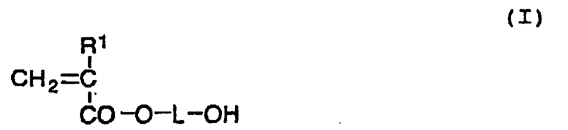

In the formula (I), L is a divalent linking group having 2 to 20 carbon atoms selected from the group consisting of a divalent aliphatic group, a divalent aromatic group and a combination of two or more divalent aliphatic or aromatic groups combined with -O-, -S-, -CO-, -NH- or a combination thereof (e.g., -O-CO-, -CO-O-, -s-co-, -CO-S-, -O-NH-, -NH-CO-, -CO-NH-, -NE-CO-O-, -O-CO-NH-, -NH-CO-NH-).

L preferably has 2 to 12 carbon atoms, and more preferably has 2 to 10 carbon atoms. The present invention is particularly effective in the case that L has 2 or 4 carbon atoms, since it was difficult according to a conventional process to synthesize a compound of the formula (V) wherein L has 2 or 4 carbon atoms.

In the present specification, the divalent aliphatic group means an alkylene group, a substituted alkylene group, an alkenylene group, a substituted alkenylene group, an alkynylene group or a substituted alkynylene group.

The alkylene group preferably has a chain structure rather than a cyclic structure. The alkylene group of the chain structure can be branched.

The alkylene moiety of the substituted alkylene group is the same as the above-described alkylene group. Examples of the substituent groups of the substituted alkylene groups include a halogen atom, cyano, hydroxyl, an aromatic group and -O-R. R is an aliphatic group or an aromatic group. The aliphatic group and the aromatic group are described below about R².

The alkenylene group preferably has a chain structure rather than a cyclic structure. The alkenylene group of the chain structure can be branched.

The alkenylene moiety of the substituted alkenylene group is the same as the above-described alkenylene group. The substituent group of the substituted alkenylene group is the same as the above-described substituent group of the substituted alkylene group.

The alkynylene group preferably has a chain structure rather than a cyclic structure. The alkynylene group of the chain structure can be branched.

The alkynylene moiety of the substituted alkynylene group is the same as the above-described alkynylene group. The substituent group of the substituted alkynylene group is the same as the above-described substituent group of the substituted alkylene group.

In the present specification, the divalent aromatic group means an arylene group or a substituted arylene group.

The arylene group preferably is phenylene or naphthylene, more preferably is phenylene, and most preferably is p-phenylene.

The arylene moiety of the substituted arylene group is the same as the above-described arylene group. Examples of the substituent groups of the substituted arylene groups include a halogen atom, cyano, hydroxyl, an aliphatic group, an aromatic group and -O-R. R is an aliphatic group or an aromatic group. The aliphatic group and the aromatic group are described below about R².

L preferably is a divalent linking group having 2 to 20 carbon atoms selected from the group consisting of a divalent aliphatic group and a combination of two or more divalent aliphatic groups combined with -O- or -S-, more preferably is an alkylene group having 2 to 20 carbon atoms or a divalent linking group having 2 to 20 carbon atoms made of two alkylene groups combined with -O-, and most preferably is an alkylene group having 2 to 20 carbon atoms.

In the formula (I), R¹ is hydrogen or methyl. R¹ preferably is hydrogen.

The (meth)acrylic ester represented by the formula (I) reacts with a sulfonyl donor at the first step. The sulfonyl donor contains an aliphatic or aromatic sulfonyl group, which can react with hydroxyl at the end of the (meth)acrylic ester represented by the formula (I) to form a sulfonic ester.

The sulfonyl donor preferably is a sulfonyl halide represented by the formula (VI-a) or a sulfonic anhydride represented by the formula (VI-b), and more preferably is the sulfonyl halide.

X-SO₂-R² (VI-a)

R²-SO₂-SO₂-R² (VI-b)

In the formula (VI-a), X is a halogen atom (F, Cl, Br, I). X preferably is chloride (Cl).

In the formulas (VI-a) and (VI-b), R² is an aliphatic group or an aromatic group.

In the present specification, the aliphatic group means an alkyl group, a substituted alkyl group, an alkenyl group, a substituted alkenyl group, an alkynyl group or a substituted alkynyl group.

The alkyl group preferably has a chain structure rather than a cyclic structure. The alkyl group of the chain structure can be branched.

The alkyl moiety of the substituted alkyl group is the same as the above-described alkyl group. Examples of the substituent groups of the substituted alkyl groups include a halogen atom, cyano, hydroxyl, an aromatic group and -O-R. R is an aliphatic group or an aromatic group.

The alkenyl group preferably has a chain structure rather than a cyclic structure. The alkenyl group of the chain structure can be branched.

The alkenyl moiety of the substituted alkenyl group is the same as the above-described alkenyl group. The substituent group of the substituted alkenyl group is the same as the above-described substituent group of the substituted alkyl group.

The alkynyl group preferably has a chain structure rather than a cyclic structure. The alkynyl group of the chain structure can be branched.

The alkynyl moiety of the substituted alkynyl group is the same as the above-described alkynyl group. The substituent group of the substituted alkynyl group is the same as the above-described substituent group of the substituted alkyl group.

In the present specification, the aromatic group means an aryl group or a substituted aryl group.

The aryl group preferably is phenyl or naphthyl, and more preferably is phenyl.

The aryl moiety of the substituted aryl group is the same as the above-described aryl group. Examples of the substituent groups of the substituted aryl groups include a halogen atom, cyano, hydroxyl, an aliphatic group, an aromatic group and -O-R. R is an aliphatic group or an aromatic group.

R² preferably is an aliphatic group, more preferably is an alkyl group, a substituted alkyl group, an alkenyl group or a substituted alkenyl group, further preferably is an alkyl group or a substituted alkyl group, and most preferably is an alkyl group. The alkyl group preferably has 1 to 10 carbon atoms, more preferably has 1 to 6 carbon atoms, further preferably has 1 to 4 carbon atoms, furthermore preferably has 1 (methyl) or 2 (ethyl) carbon atoms, and most preferably has 1 carbon atom (methyl).

At the first step, the (meth)acrylic ester represented by the formula (I) reacts with the sulfonyl donor to synthesize a sulfonic ester represented by the formula (II).

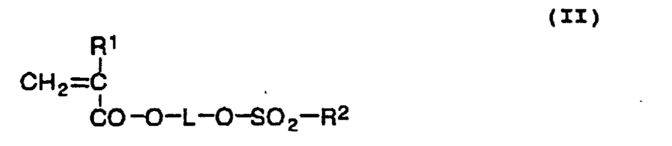

In the formula (II), L, R¹ and R² have the same meanings as defined in the formulas (I), (VI-a) and (VI-b).

In the case that the sulfonyl donor is a sulfonyl halide represented by the formula (VI-a), the first step is represented by the following reaction formula.

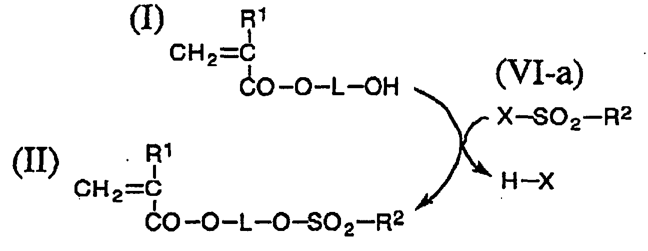

In the reaction formula, L, X, R¹ and R² have the same meanings as defined in the formulas (I) and (VI-a).

In the case that the sulfonyl donor is a sulfonic anhydride represented by the formula (VI-b), the first step is represented by the following reaction formula.

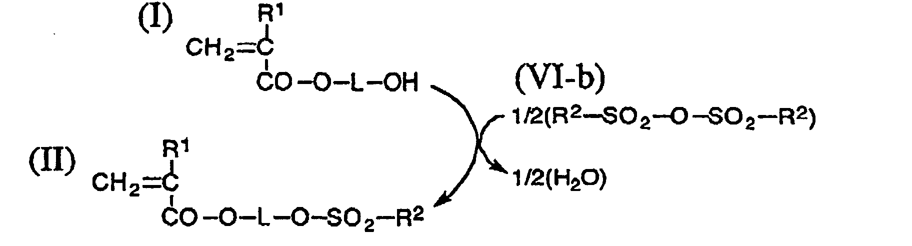

In the reaction formula, L, R¹ and R² have the same meanings as defined in the formulas (I) and (VI-b).

A reaction of the first step wherein R² is tolyl is described in New Course of Experimental Chemistry (written in Japanese), vol. 14, Synthesis and Reaction of Organic Compound III, Maruzen, 1977, pp. 1,793. A reaction of the first step wherein R² is methyl is described in the above book, pp. 1,797.

The first step is preferably conducted in an organic solvent in the presence of a base.

Examples of the organic solvent include an ester (e.g., ethyl acetate), a hydrocarbon (e.g., toluene), an ether (e.g., tetrahydrofuran), an amide (e.g., dimethylformamide, dimethylacetamide), a halogenated hydrocarbon (e.g., dichloromethane), a nitrile (e.g., acetonitrile) and a mixture thereof. The ester and the hydrocarbon are preferred, and ethyl acetate and toluene are particularly preferred.

The base can be an inorganic base (e.g., potassium carbonate) or an organic base (e.g., triethylamine). The organic base is preferred, and triethylamine is particularly preferred.

The amount of the sulfonyl donor is preferably in the range of 0.5 to 3 equivalents, and more preferably in the range of 0.8 to 1.5 equivalent based on the amount of the (meth)acrylic ester of the formula (I).

The reaction temperature of the first step is usually in the range of -20°C to the boiling point of the used solvent, and preferably in the range of 0°C to room temperature. The reaction time is usually in the range of 10 minutes to 3 days, and preferably in the range of 1 hour to 1 day.

The sulfonic ester of the formula (II) obtained at the step (I) reacts with an aromatic aldehyde of the following formula (III) at the second step.

H-Y-Ar-CHO (III)

In the formula (III), Y is oxygen or sulfur. Oxygen is preferred to sulfur.

In the formula (III), Ar is a divalent aromatic group. The divalent aromatic group is described above about the formula (I).

Ar preferably is phenylene or a substituted phenylene group, and more preferably is p-phenylene or a substituted p-phenylene group.

The substituent group of the divalent aromatic group preferably is an inert group (such as an alkyl group, an alkoxy group, a halogen atom) at the second and third steps.

At the second step, the sulfonic ester of the formula (II) obtained at the step (I) reacts with the aromatic aldehyde of the formula (III) to synthesize an aromatic aldehyde of the formula (IV).

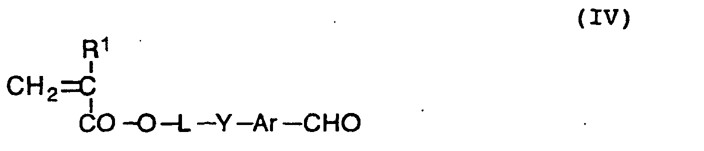

In the formula (IV), L, R¹, Y and Ar have the same meanings as defined in the formulas (II) and (III).

The second step is represented by the following reaction formula.

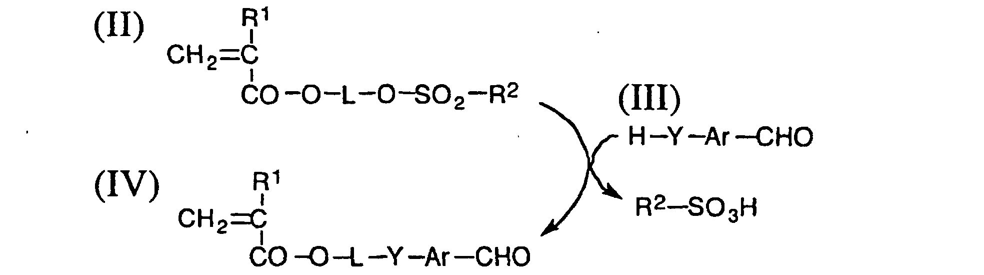

In the reaction formula, L, R¹, R², Y and Ar have the same meanings as defined in the formulas (II) and (III).

The second step is preferably conducted in an organic solvent in the presence of a base.

Examples of the organic solvent include an ester (e.g., ethyl acetate), a hydrocarbon (e.g., toluene), an ether (e.g., tetrahydrofuran), an amide (e.g., dimethylformamide, dimethylacetamide), a halogenated hydrocarbon (e.g., dichloromethane), a nitrile (e.g., acetonitrile) and a mixture thereof. The ester, the hydrocarbon, the amide and a mixture thereof are preferred, and ethyl acetate, toluene, dimethylformamide, dimethylacetamide and a mixture thereof are more preferred.

The base can be an inorganic base (e.g., potassium carbonate) or an organic base (e.g., triethylamine). The inorganic base is preferred, and potassium carbonate is particularly preferred.

The amount of the aromatic aldehyde of the formula (III) is preferably in the range of 0.5 to 3 equivalents, and more preferably in the range of 0.8 to 1.5 equivalent based on the amount of the sulfonic ester of the formula (II).

The reaction temperature of the second step is usually in the range of -20°C to the boiling point of the used solvent, and preferably not lower than 80°C. The reaction time is usually in the range of 10 minutes to 3 days, and preferably in the range of 1 hour to 1 day.

The aromatic aldehyde of the formula (IV) is oxidized to synthesize an aromatic carboxylic acid represented by the following formula (V) at the third step.

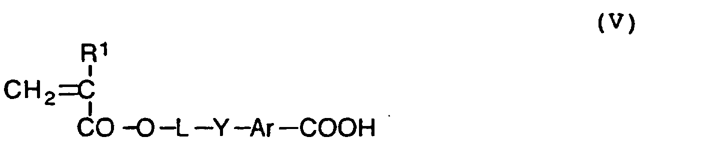

In the formula (V), L, R¹, Y and Ar have the same meanings as defined in the formulas (II) and (III).

The third step is represented by the following reaction formula.

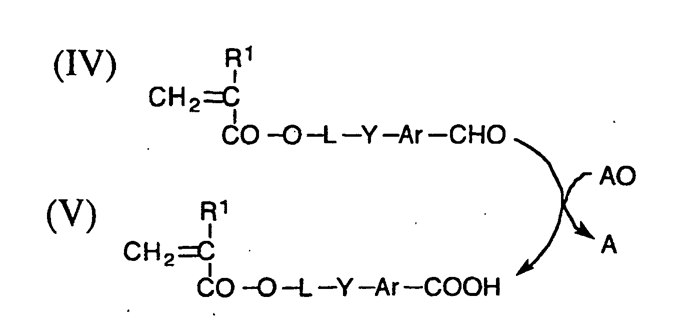

In the reaction formula, L, R¹, Y and Ar have the same meanings as defined in the formulas (II) and (III). AO is an oxidizing agent, and A is a reduced form of the oxidizing agent (after the oxidation reaction).

The oxidizing agent (AO) preferably is inert to the double bond of the (meth)acryloyloxy group. Examples of the oxidizing agents include chlorite salt and perborate salt. The chlorite salt is preferred, an alkali metal chlorite salt is more preferred, and sodium chlorite is most preferred.

The third step is preferably conducted in an organic solvent while adjusting the reaction pH.

Examples of the organic solvent include an ester (e.g., ethyl acetate), a hydrocarbon (e.g., toluene), an ether (e.g., tetrahydrofuran), an amide (e.g., dimethylformamide, dimethylacetamide), a halogenated hydrocarbon (e.g., dichloromethane), a nitrile (e.g., acetonitrile) and a mixture thereof. The ester, the amide, the nitrile and a mixture thereof are preferred, and ethyl acetate, dimethylformamide, dimethylacetamide, acetonitrile and a mixture thereof are more preferred.

The reaction pH is preferably adjusted to about 4. The pH is adjusted preferably by using a phosphate buffer. The reaction at the third step is preferably conducted in a mixture of the organic solvent and an aqueous solution of sodium dihydrogenphosphate.

In the case that chlorite salt is used as the oxidizing agent, hydrogen peroxide is preferably added to the reaction system. The reaction of the third step using a chlorite salt and hydrogen peroxide is shown below.

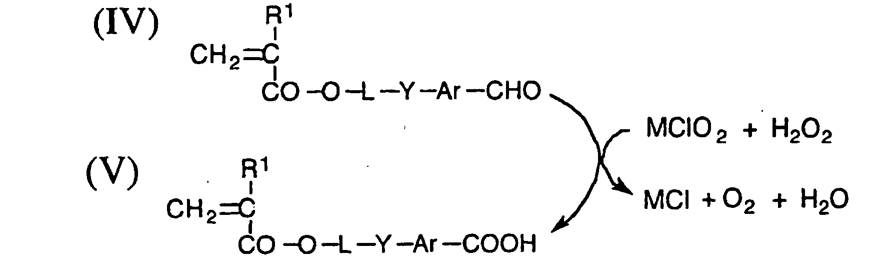

In the reaction formula, L, R¹, Y and Ar have the same meanings as defined in the formulas (II) and (III). M is a counter ion of the chlorite salt.

The hydrogen peroxide preferably in the form of an aqueous solution is preferably added to the reaction system. The concentration of the aqueous hydrogen peroxide solution is preferably in the range of 0.1 to 50 wt.%, and more preferably in the range of 3 to 40 wt.%.

The amount of the oxidizing agent is preferably in the range of 0.5 to 10 equivalents, and more preferably in the range of 0.8 to 5 equivalents based on the amount of the aromatic aldehyde of the formula (IV). In the case that the hydrogen peroxide is used, the amount of the hydrogen peroxide is preferably in the range of 0.5 to 50 equivalents, and more preferably in the range of 3 to 20 equivalents based on the amount of the aromatic aldehyde of the formula (IV).

The reaction temperature of the third step is usually in the range of -20°C to the boiling point of the used solvent, and preferably in the range of 0°C to room temperature. The reaction time is usually in the range of 10 minutes to 3 days, and preferably in the range of 1 hour to 1 day.

At least one step is preferably conducted in the presence of a polymerization inhibitor. More preferably at least third step, further preferably the second and third steps, and most preferably all steps are conducted in the presence of a polymerization inhibitor. In the case that the first to third steps are conducted in a reaction vessel successively, the polymerization inhibitor is preferably added to the reaction vessel at each of the steps.

Examples of the polymerization inhibitors include a condensed aromatic heterocyclic compound (e.g., phenothiazine), a condensed aromatic hydrocarbon compound (e.g., fluorene), a nitro-substituted aromatic hydrocarbon compound (e.g., nitrobenzene, nitrophenol), a halogen-substituted aromatic hydrocarbon compound (e.g., chlorobenzene) and a hydroquinone monoalkyl ether (e.g., hydroquinone monomethyl ether). The nitro-substituted aromatic hydrocarbon compound and the hydroquinone monoalkyl ether are preferred, and nitrobenzene and hydroquinone monomethyl ether is particularly preferred.

The sulfonic ester represented by the formula (II) is preferably not purified and used at the step of synthesizing the aromatic aldehyde represented by the formula (IV). The aromatic aldehyde represented by the formula (IV) is also preferably not purified and used at the step of synthesizing the aromatic carboxylic acid represented by the formula (V). In other words, the first step (of synthesizing the sulfonic ester represented by the formula (II)), the second step (of synthesizing the aromatic aldehyde represented by the formula (IV)) and the third step of synthesizing the aromatic carboxylic acid represented by the formula (V)) are preferably conducted continuously. An aromatic carboxylic acid can be prepared in a high yield where the first to third steps are continuously conducted, as is described above.

The first to third steps can be conducted in a reaction vessel successively. In more detail, a sulfonyl donor (the first step), an aromatic aldehyde represented by the formula (III) (the second step) and an oxidizing agent (the third step) can be successively added to a reaction vessel containing a (meth)acrylic ester represented by the formula (I) to proceed the reactions continuously.

### EXAMPLE 1

### Preparation of 4-(4-acryloyloxybutyloxy)benzoic acid

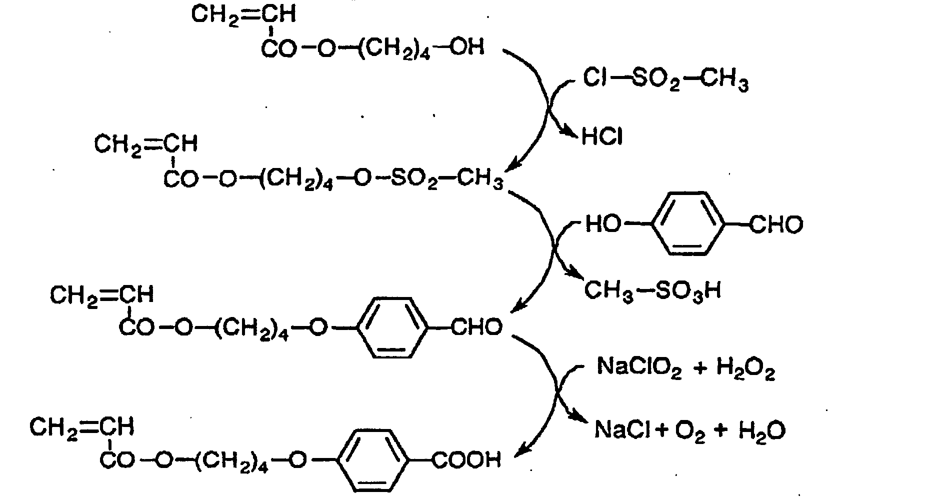

In 30 mL of ethyl acetate, 5.0 g of 4-acryloyloxybutyl alcohol and 5.3 mL of triethylamine were dissolved. While the solution was cooled with ice, 3.77 g of mesyl chloride dissolved in 5 mL of ethyl acetate was dropwise added. The reaction liquid was stirred for 30 minutes at room temperature, and then washed with 20 mL of 0.2 N hydrochloric acid.

To the solution, 4.02 g of p-hydroxybenzaldehyde dissolved in 40 mL of dimethylformamide was added, and successively 9.58 g of potassium carbonate was further added. The resulting solution was stirred for 2 hours at 100°C, and then washed with 40 mL of water.

The solution was heated and evaporated to concentrate to 40 mL, and cooled to room temperature. To the cooled liquid, 66 mL of acetonitrile, phosphate buffer solution (1.06 g of sodium dihydrogenphosphate dihydrate dissolved in 13.2 mL of water) and 4.2 mL of 35% aqueous solution of hydrogen peroxide were added, and successively 4.16 g of sodium chlorite dissolved in 33 mL of water was further added. The obtained solution was stirred for 5 hours at room temperature, and then left for 8 hours. To the solution, 90 mL of 0.1 N hydrochloric acid was added. The deposited precipitate was collected to obtain 4-(4-acryloyloxybutyloxy)benzoic acid.
- Amount:: 7.07 g
- Purity:: 99.2%
- Yield:: 81.8% (based on the amount of mesyl chloride or p-hydroxybenzaldehyde)

### EXAMPLE 2

### Preparation of 4-(24-(2-acryloyloxyethyloxy)benzoic)benzoic acid

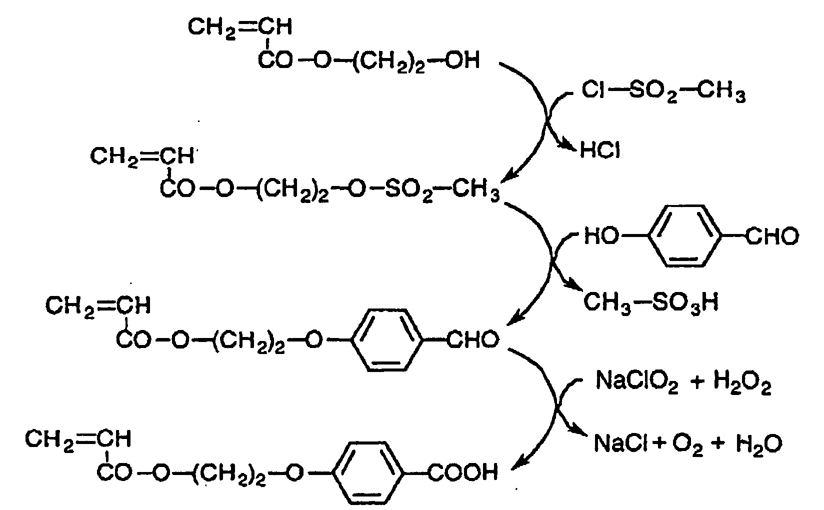

In 60 mL of ethyl acetate, 11.61 g of 2-acryloyloxyethyl alcohol and 14 mL of triethylamine were dissolved. while the solution was cooled with ice, 12.60 g of mesyl chloride dissolved in 10 mL of ethyl acetate was dropwise added. The reaction liquid was stirred for 30 minutes at room temperature, and then washed with 50 mL of 0.2 N hydrochloric acid.

To the solution, 12.21 g of p-hydroxybenzaldehyde dissolved in 100 mL of dimethylformamide was added, and successively 34.50 g of potassium carbonate was further added. The resulting solution was stirred for 4 hours at 80°C, and then washed with 100 mL of water.

The solution was heated and evaporated to concentrate to 100 mL, and cooled to room temperature. To the cooled liquid, 200 mL of acetonitrile, phosphate buffer solution (3.20 g of sodium dihydrogenphosphate dihydrate dissolved in 40.0 mL of water) and 12.5 mL of 35% aqueous solution of hydrogen peroxide were added, and successively 12.60 g of sodium chlorite dissolved in 30 mL of water was further added. The obtained solution was stirred for 6 hours at room temperature, and then left for 8 hours. To the solution, 20 mL of 0.1 N hydrochloric acid was added. The deposited precipitate was collected to obtain 4-(2-acryloyloxyethyloxy)benzoic acid.
- Amount:: 20.50 g
- Purity:: 98.7%
- Yield:: 78.9% (based on the amount of mesyl chloride)

### EXAMPLE 3

### Preparation of 4-(64-(6-acryloyloxyhexyloxy)benzoic )benzoic acid

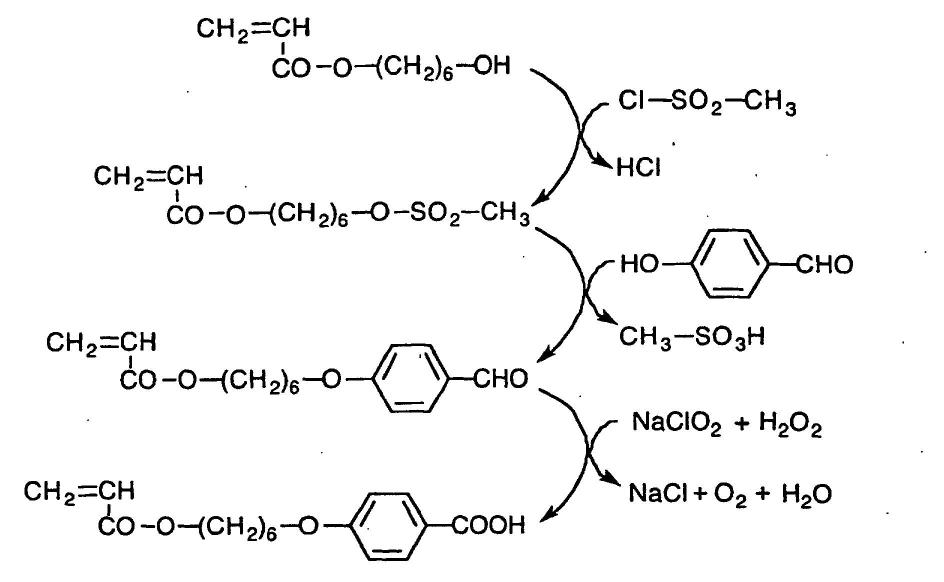

In 30 mL of ethyl acetate, 8.60 g of 6-acryloyloxyhexyl alcohol and 8.35 mL of triethylamine were dissolved. While the solution was cooled with ice, 6.30 g of mesyl chloride dissolved in 10 mL of ethyl acetate was dropwise added. The reaction liquid was stirred for 1 hour at room temperature, and then washed with 25 mL of 0.2 N hydrochloric acid.

To the solution, 6.11 g of p-hydroxybenzaldehyde dissolved in 50 mL of dimethylformamide was added, and successively 13.80 g of potassium carbonate was further added. The resulting solution was stirred for 4 hours at 100°C, and then washed with 50 mL of water.

The solution was heated and evaporated to concentrate to 50 mL, and cooled to room temperature. To the cooled liquid, 100 mL of acetonitrile, phosphate buffer solution (1.60 g of sodium dihydrogenphosphate dihydrate dissolved in 20.0 mL of water) and 6.25 mL of 35% aqueous solution of hydrogen peroxide were added, and successively 6.30 g of sodium chlorite dissolved in 10 mL of water was further added. The obtained solution was stirred for 6 hours at room temperature, and then left for 8 hours. To the solution, 10 mL of 0.1 N hydrochloric acid was added. The deposited precipitate was collected to obtain 4-(6-acryloyloxyhexyloxy)benzole acid.
- Amount:: 13.40 g
- Purity:: 98.5%
- Yield:: 83.3% (based on the amount of mesyl chloride)

### EXAMPLE 4

### Preparation of 4-(2-(2-acryloyloxyethyloxy)ethyloxy)benzoic acid

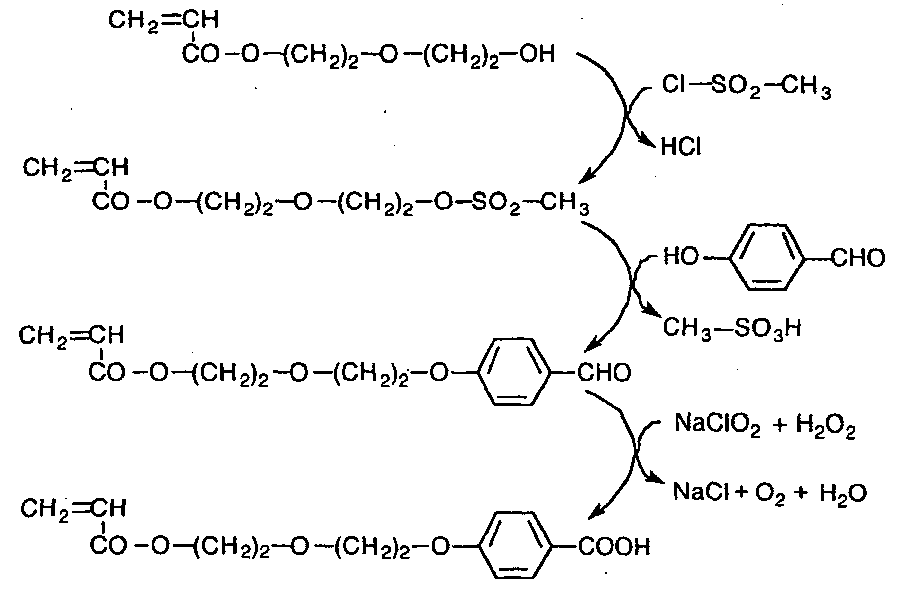

In 60 mL of ethyl acetate, 16.01 g of 2-(2-acryloyloxyethyloxyjethyl alcohol and 14 mL of triethylamine were dissolved. While the solution was cooled with ice, 12.60 g of mesyl chloride dissolved in 10 mL of ethyl acetate was dropwise added. The reaction liquid was stirred for 30 minutes at room temperature, and then washed with 50 mL of 0.2 N hydrochloric acid.

To the solution, 12.21 g of p-hydroxybenzaldehyde dissolved in 100 mL of dimethylformamide was added, and successively 34.50 g of potassium carbonate was further added. The resulting solution was stirred for 4 hours at 80°C, and then washed with 100 mL of water.

The solution was heated and evaporated to concentrate to 100 mL, and cooled to room temperature. To the cooled liquid, 200 mL of acetonitrile, phosphate buffer solution (3.20 g of sodium dihydrogenphosphate dihydrate dissolved in 40.0 mL of water) and 25.0 mL of 35% aqueous solution of hydrogen peroxide were added, and successively 12.60 g of sodium chlorite dissolved in 30 mL of water was further added. The obtained solution was stirred for 6 hours at room temperature, and then left for 8 hours. The solution was concentrated to 100 mL under a reduced pressure. To the solution, 50 mL of 0.1 N hydrochloric acid was added. The deposited precipitate was collected to obtain 4-(2-(2-acryloyloxyethyloxy)ethyloxy)benzoic acid.
- Amount:: 23.55 g
- Purity:: 96.4%
- Yield:: 76.4% (based on the amount of mesyl chloride)

### EXAMPLE 5

### Preparation of 4-(44-(4-methacryloyloxybutyloxy)benzoic )benzoic acid

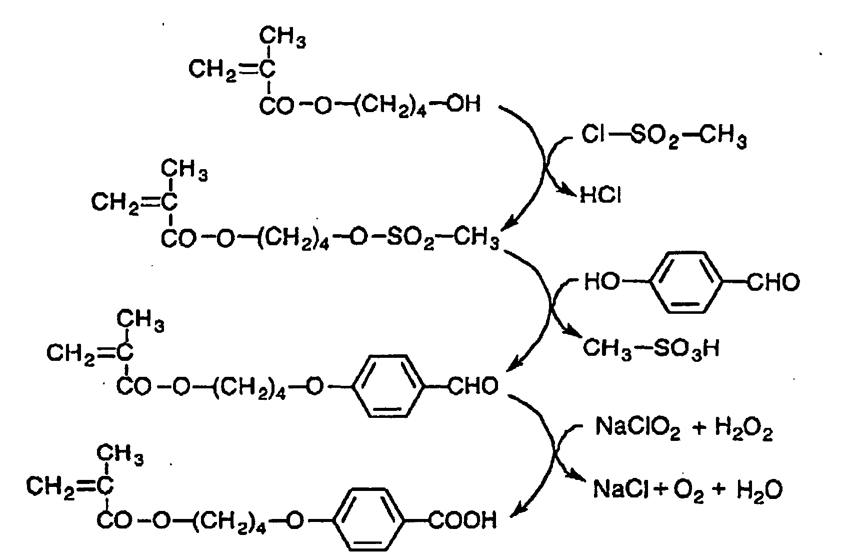

In 60 mL of ethyl acetate, 15.82 g of 4-methacryloyloxybutyl alcohol and 14 mL of triethylamine were dissolved. While the solution was cooled with ice, 12.60 g of mesyl chloride dissolved in 10 mL of ethyl acetate was dropwise added. The reaction liquid was stirred for 30 minutes at room temperature, and then washed with 50 mL of 0.2 N hydrochloric acid.

To the solution, 12.21 g of p-hydroxybenzaldehyde dissolved in 100 mL of dimethylformamide was added, and successively 34.50 g of potassium carbonate was further added. The resulting solution was stirred for 8 hours at 80°C, and then washed with 100 mL of water.

The solution was heated and evaporated to concentrate to 100 mL, and cooled to room temperature. To the cooled liquid, 200 mL of acetonitrile, phosphate buffer solution (3.20 g of sodium dihydrogenphosphate dihydrate dissolved in 40.0 mL of water) and 12.5 mL of 35% aqueous solution of hydrogen peroxide were added, and successively 12.60 g of sodium chlorite dissolved in 30 mL of water was further added. The obtained solution was stirred for 6 hours at room temperature, and then left for 8 hours. To the solution, 50 mL of 0.1 N hydrochloric acid was added. The deposited precipitate was collected to obtain 4-(4-methacryloyloxybutyloxy)benzoic acid.
- Amount:: 25.87 g
- Purity:: 98.9%
- Yield:: 84.5% (based on the amount of mesyl chloride)

### EXAMPLE 6

### Preparation of 4-(34-(3-methacryloyloxypropyloxy)benzoic acid

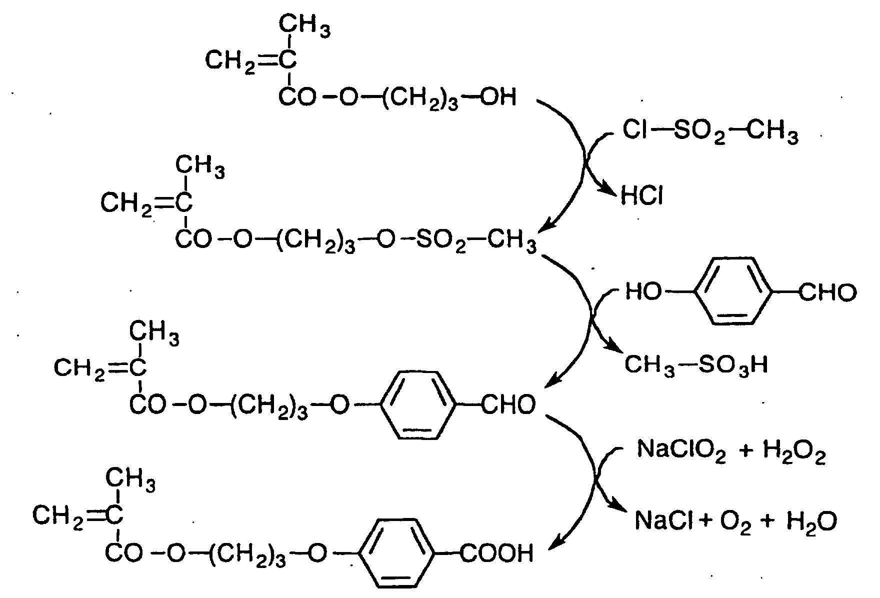

The procedure of Example 5 was repeated except that 3-methacryloyloxypropyl alcohol (14.42 g) was used in place of 4-methacryloyloxybutyl alcohol (15.82 g), to prepare 4-(3-methacryloyloxypropyloxy)benzoic acid.
- Purity:: 98.2%
- Yield:: 82.8% (based on the amount of mesyl chloride)

### EXAMPLE 7

### Preparation of 3-(43-(4-acryloyloxybutyloxy)benzoic)benzoic acid

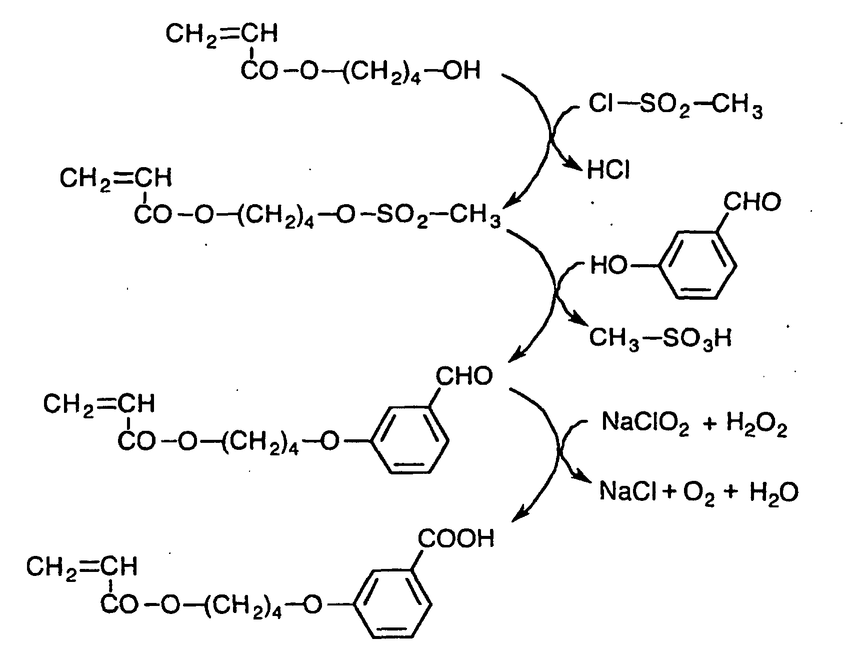

The procedure of Example 1 was repeated except that m-hydroxybenzaldehyde was used in place of p-hydroxybenzaldehyde, to prepare 3-(4-acryloyloxybutyloxy)benzoic acid.
- Purity:: 97.6%
- Yield:: 82.3% (based on the amount of mesyl chloride)

### EXAMPLE 8

### Preparation of 4-(4-acryloyloxybutyloxy)-3-methoxybenzoic acid

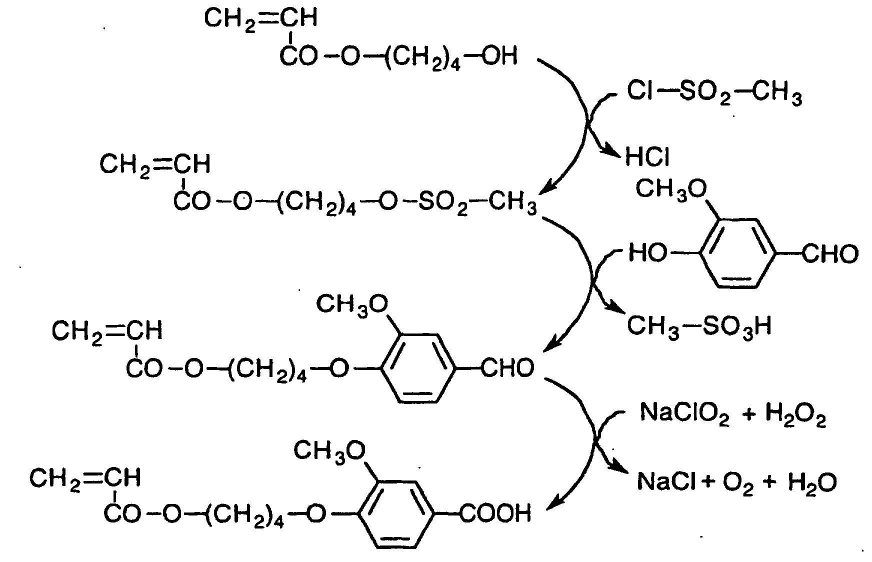

In 60 mL of ethyl acetate, 14.42 g of 4-acryloyloxybutyl alcohol, 0.12 g of nitrobenzene and 14 mL of triethylamine were dissolved. While the solution was cooled with ice, 12.60 g of mesyl chloride dissolved in 10 mL of ethyl acetate was dropwise added. The reaction liquid was stirred for 1 hour at room temperature, and then washed with 50 mL of 0.2 N hydrochloric acid.

To the solution, 15.22 g of vanillin dissolved in 100 mL of dimethylformamide was added, and successively 34.50 g of potassium carbonate was further added. The resulting solution was stirred for 4 hours at 80°C, and then washed with 100 mL of water.

The solution was heated and evaporated to concentrate to 100 mL, and cooled to room temperature. To the cooled liquid, 200 mL of acetonitrile, phosphate buffer solution (3.20 g of sodium dihydrogenphosphate dihydrate dissolved in 40.0 mL of water) and 12.5 mL of 35% aqueous solution of hydrogen peroxide were added, and successively 12.60 g of sodium chlorite dissolved in 30 mL of water was further added. The obtained solution was stirred for 6 hours at room temperature, and then left for 8 hours. To the solution, 20 mL of 0.1 N hydrochloric acid was added. The deposited precipitate was collected to obtain 4-(4-acryloyloxybutyloxy)-3-methoxybenzoic acid.
- Amount:: 23.64 g
- Purity:: 97.8%
- Yield:: 73.0% (based on the amount of mesyl chloride)

### EXAMPLE 9

### Preparation of 4-(4-acryloyloxybutyloxy)-3-methylbenzoic acid

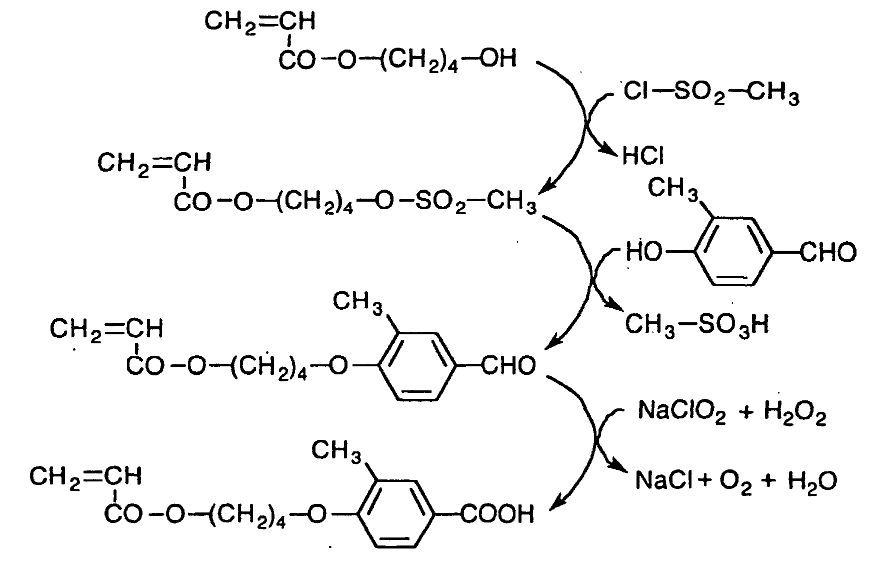

The procedure of Example 8 was repeated except that 13.62 g of 4-hydroxy-3-methylbenzaldehyde was used in place of 15.22 g of vanillin, to prepare 4-(4-acryloyloxybutyloxy)-3-methylbenzoic acid
- Purity:: 97.9%
- Yield:: 76.2% (based on the amount of mesyl chloride)

### COMPARISON EXAMPLE 1

### Preparation of 4-(2-acryloyloxyethyloxy)benzoic acid

According to a method disclosed in Japanese Patent Provisional Publication No. 59(1984)-70643, 4-(2-acryloyloxyethyloxy)benzoic acid was prepared.
Yield: 31.2% (based on the amount of p-hydroxybenzaldehyde)

### COMPARISON EXAMPLE 2

### Preparation of 4-(4-acryloyloxybutyloxy)benzoic acid

According to a method disclosed in Japanese Patent Provisional Publication No. 59(1984)-70643, 4-(4-acryloyloxybutyloxy)benzoic acid was prepared.
Yield: 29.8% (based on the amount of p-hydroxybenzaldehyde)

### COMPARISON EXAMPLE 3

### Preparation of 4-(4-acryloyloxybutyloxy)benzaldehyde

According to a method disclosed in U.S. Patent No. 5,087,672, 4-(4-acryloyloxybutyloxy)benzaldehyde, which is an intermediate product for 4-(4-acryloyloxybutyloxy)benzoic acid, was prepared from 4-chloro-1-butanol and p-hydroxybenzaldehyde (starting materials).
Yield: 11.7% (based on the amount of p-hydroxybenzaldehyde)

### EXAMPLE 10

### Process for preparation of 4-(4-acryloyloxybutyloxy)benzoic acid

In a reaction pot, 130 Kg of 4-acryloyloxybutyl alcohol, 0.54 Kg of nitrobenzene and 765 liters of ethyl acetate were placed. To the mixture, 114 Kg of triethylamine was added. To the resulting mixture, 110 Kg of methanesulfonyl chloride was dropwise added at 0°C. After completing the reaction, 630 liters of water was added to the mixture, and separated.

Separately, 99 Kg of p-hydroxybenzaldehyde, 94 Kg of potassium carbonate, 0.54 Kg of nitrobenzene and 500 liters of dimethylformamide were placed in another reaction pot to form a solution. The above-prepared reaction solution was added to the pot. The mixture was heated at 90°C and stirred for 5 hours. After the mixture was cooled, 1,080 liters of water was added to the mixture. The resulting mixture was separated. The organic phase was washed with 720 liters of water, left at 25°C over one night, and separated. To the separated, 0.54 Kg of hydroquinone monomethyl ether and 480 liters of acetonitrile were added. The resulting mixture was stirred. Further, 540 liters of water, 275 liters of 9.1 wt.% aqueous solution of mono sodium phosphate, and 184 Kg of 30 wt.% aqueous solution of hydrogen peroxide were added to the mixture in this order. To the mixture, 380 Kg of 25 wt.% aqueous solution of sodium hydrogen chlorite was dropwise added. The mixture was stirred for 4 hours. After completing the reaction, 1,800 liters of water was added to the mixture. The mixture was subjected to a centrifugal filtration to obtain crude 4-(4-acryloyloxybutyloxy)benzoic acid as white crystals.

### Process for purification of 4-(4-acryloyloxybutyloxy)benzoic acid

In a reaction bath, 1,300 liters of acetonitrile, 172 liters of water and 4 Kg of concentrated sulfuric acid were placed. To the mixture, the obtained crude 4-(4-acryloyloxybutyloxy)benzoic acid was added. The mixture was heated to 70°C to obtain a solution. The solution was subjected to a dustproof filtration, and was recrystallized. The obtained white crystals were separated by a centrifugal filtration, and dried to obtain 131 Kg (yield: 61%) of 4-(4-acryloyloxybutyloxy)benzoic acid.

## Claims

1. A process for the preparation of an aromatic carboxylic acid represented by the formula (V), which comprises the steps of: synthesizing a sulfonic ester represented by the formula (II) by a reaction of a (meth)acrylic ester represented by the formula (I) with a sulfonyl donor; synthesizing an aromatic aldehyde represented by the formula (IV) by a reaction of the sulfonic ester represented by the formula (II) with an aromatic aldehyde represented by the formula (III); and synthesizing the aromatic carboxylic acid represented by the formula (V) by an oxidation of the aromatic aldehyde represented by the formula (IV): 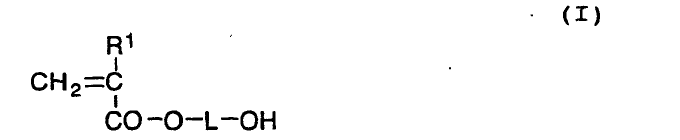 in which L is a divalent linking group having 2 to 20 carbon atoms selected from the group consisting of a divalent aliphatic group, a divalent aromatic group and a combination of two or more divalent aliphatic or aromatic groups combined with -O-, -S-, -CO-, -NH- or a combination thereof; and R¹ is hydrogen or methyl; 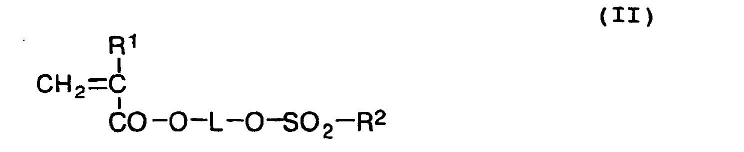 in which L is a divalent linking group having 2 to 20 carbon atoms selected from the group consisting of a divalent aliphatic group, a divalent aromatic group and a combination of two or more divalent aliphatic or aromatic groups combined with -O-, -S-, -CO-, -NH- or a combination thereof; R¹ is hydrogen or methyl; and R² is an aliphatic group or an aromatic group;
H-Y-Ar-CHO (III)
in which Y is oxygen or sulfur; and Ar is a divalent aromatic group; 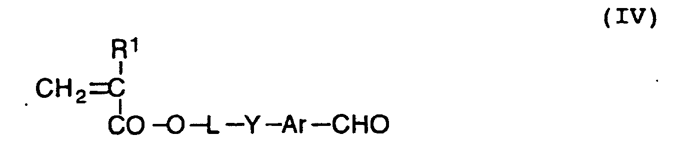 in which L is a divalent linking group having 2 to 20 carbon atoms selected from the group consisting of a divalent aliphatic group, a divalent aromatic group and a combination of two or more divalent aliphatic or aromatic groups combined with -O-, -S-, -CO-, -NH- or a combination thereof; R¹ is hydrogen or methyl; Y is oxygen or sulfur; and Ar is a divalent aromatic group; 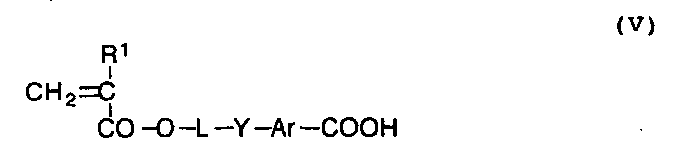 in which L is a divalent linking group having 2 to 20 carbon atoms selected from the group consisting of a divalent aliphatic group, a divalent aromatic group and a combination of two or more divalent aliphatic or aromatic groups combined with -O-, -S-, -CO-, -NH- or a combination thereof; R¹ is hydrogen or methyl; Y is oxygen or sulfur; and Ar is a divalent aromatic group.

2. The process as claimed in claim 1, wherein L in the formulas (I), (II), (IV) and (V) is a divalent linking group having 2 to 20 carbon atoms selected from the group consisting of a divalent aliphatic group and a combination of two or more divalent aliphatic groups combined with -O- or -S-.

3. The process as claimed in claim 1, wherein L in the formulas (I), (II), (IV) and (V) is a divalent linking group having 2 or 4 carbon.

4. The process as claimed in claim 1, wherein the sulfonyl donor is a sulfonyl halide represented by the formula (VI-a) or a sulfonic anhydride represented by the formula (VI-b):
X-SO₂-R² (VI-a)
R²-SO₂-O-SO₂-R² (VI-b)
in which X is a halogen atom; and R² is an aliphatic group or an aromatic group.

5. The process as claimed in claim 4, wherein R² is an alkyl group.

6. The process as claimed in claim 1, wherein Y in the formulas (III), (IV) and (V) is oxygen.

7. The process as claimed in claim 1, wherein Ar in the formulas (III), (IV) and (V) is p-phenylene or a substituted p-phenylene group.

8. The process as claimed in claim 1, wherein the aromatic aldehyde is oxidized by using a chlorite salt as an oxidizing agent.

9. The process as claimed in claim 1, wherein at least one step is conducted in the presence of a polymerization inhibitor.

10. A process for the preparation of an aromatic aldehyde represented by the formula (IV), which comprises the steps of: synthesizing a sulfonic ester represented by the formula (II) by a reaction of a (meth)acrylic ester represented by the formula (I) with a sulfonyl donor; and synthesizing the aromatic aldehyde represented by the formula (IV) by a reaction of the sulfonic ester represented by the formula (II) with an aromatic aldehyde represented by the formula (III): 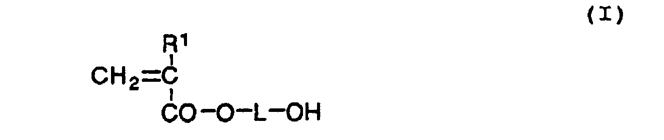 in which L is a divalent linking group having 2 to 20 carbon atoms selected from the group consisting of a divalent aliphatic group, a divalent aromatic group and a combination of two or more divalent aliphatic or aromatic groups combined with -O-, -S-, -CO-, -NH- or a combination thereof; and R¹ is hydrogen or methyl; 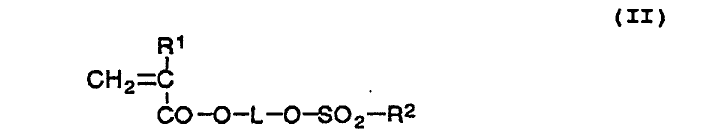 in which L is a divalent linking group having 2 to 20 carbon atoms selected from the group consisting of a divalent aliphatic group, a divalent aromatic group and a combination of two or more divalent aliphatic or aromatic groups combined with -O-, -S-, -CO-, -NH- or a combination thereof; R¹ is hydrogen or methyl; and R² is an aliphatic group or an aromatic group;
H-Y-Ar-CHO (III)
in which Y is oxygen or sulfur; and Ar is a divalent aromatic group; 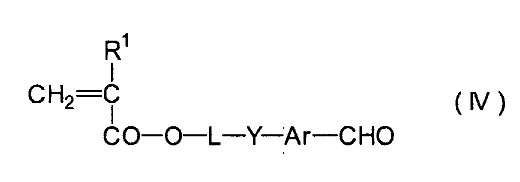 in which L is a divalent linking group having 2 to 20 carbon atoms selected from the group consisting of a divalent aliphatic group, a divalent aromatic group and a combination of two or more divalent aliphatic or aromatic groups combined with -O-, -S-, -CO-, -NH- or a combination thereof; R¹ is hydrogen or methyl; Y is oxygen or sulfur; and Ar is a divalent aromatic group.

## Patentansprüche

1. Verfahren zur Herstellung einer aromatischen Carbonsäure, dargestellt durch Formel (V), das die folgenden Schritte umfasst: Synthese eines Sulfonsäureesters, dargestellt durch Formel (II), durch Umsetzung eines (Meth)acrylsäureesters, dargestellt durch Formel (I), mit einem Sulfonyldonor; Synthese eines aromatischen Aldehyds, dargestellt durch Formel (IV), durch Umsetzung eines Sulfonsäureesters, dargestellt durch Formel (II), mit einem aromatischen Aldehyd, dargestellt durch Formel (III); und Synthese der aromatischen Carbonsäure, dargestellt durch Formel (V), durch Oxidation des aromatischen Aldehyds, dargestellt durch Formel (IV): 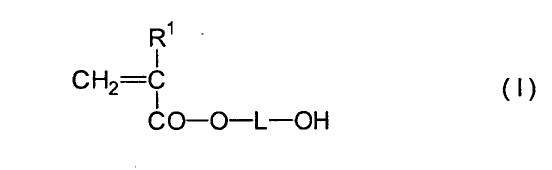 worin L eine divalente Bindungsgruppe mit 2 bis 20 Kohlenstoffatomen ist, ausgewählt aus der Gruppe, bestehend aus einer divalenten, aliphatischen Gruppe, einer divalenten, aromatischen Gruppe und einer Kombination von zwei oder mehreren divalenten, aliphatischen oder aromatischen Gruppen, kombiniert mit -O-, -S-, -CO-, -NH- oder einer Kombination davon; und R¹ ist Wasserstoff oder Methyl; 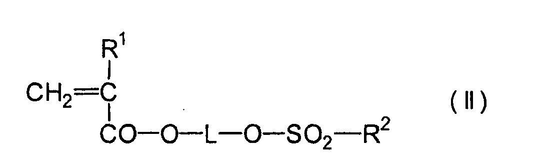 worin L eine divalente Bindungsgruppe mit 2 bis 20 Kohlenstoffatomen ist, ausgewählt aus der Gruppe, bestehend aus einer divalenten, aliphatischen Gruppe, einer divalenten, aromatischen Gruppe und einer Kombination von zwei oder mehreren divalenten, aliphatischen oder aromatischen Gruppen, kombiniert mit -O-, -S-, -CO-, -NH- oder einer Kombination davon; R¹ ist Wasserstoff oder Methyl und R² ist eine aliphatische Gruppe oder eine aromatische Gruppe;
H-Y-Ar-CHO (III)
worin Y Sauerstoff oder Schwefel ist und Ar eine divalente, aromatische Gruppe ist; 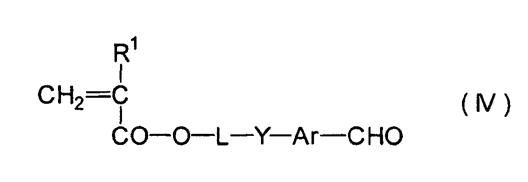 worin L eine divalente Bindungsgruppe mit 2 bis 20 Kohlenstoffatomen ist, ausgewählt aus der Gruppe, bestehend aus einer divalenten, aliphatischen Gruppe, einer divalenten, aromatischen Gruppe und einer Kombination von zwei oder mehreren divalenten, aliphatischen oder aromatischen Gruppen, kombiniert mit -O-, -S-, -CO-, -NH- oder einer Kombination davon; und R¹ ist Wasserstoff oder Methyl; Y ist Sauerstoff oder Schwefel und Ar ist eine divalente, aromatische Gruppe; 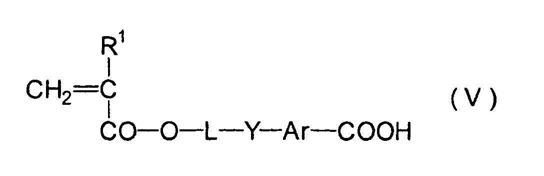 worin L eine divalente Bindungsgruppe mit 2 bis 20 Kohlenstoffatomen ist, ausgewählt aus der Gruppe, bestehend aus einer divalenten, aliphatischen Gruppe, einer divalenten, aromatischen Gruppe und einer Kombination von zwei oder mehreren divalenten, aliphatischen oder aromatischen Gruppen, kombiniert mit -O-, -S-, -CO-, -NH- oder einer Kombination davon; R¹ ist Wasserstoff oder Methyl; Y ist Sauerstoff oder Schwefel und Ar ist eine divalente, aromatische Gruppe.

2. Verfahren gemäss Anspruch 1, worin L in den Formeln (I), (II), (IV) und (V) eine divalente Bindungsgruppe mit 2 bis 20 Kohlenstoffatomen ist, ausgewählt aus der Gruppe, bestehend aus einer divalenten, aliphatischen Gruppe und einer Kombination von zwei oder mehreren divalenten, aliphatischen Gruppen, kombiniert mit -O- oder -S-.

3. Verfahren gemäss Anspruch 1, worin L in den Formeln (I), (II), (IV) und (V) eine divalente Bindungsgruppe mit 2 bis 4 Kohlenstoffatomen ist.

4. Verfahren gemäss Anspruch 1, worin der Sulfonyldonor ein Sulfonylhalogenid ist, dargestellt durch die Formel (VI-a), oder ein Sulfonsäureanhydrid, dargestellt durch die Formel (VI-b):
X-SO₂-R² (VI-a)
R² -SO₂-O-SO₂-R² (VI-b)
worin X ein Halogenatom ist und R² eine aliphatische Gruppe oder eine aromatische Gruppe ist.

5. Verfahren gemäss Anspruch 4, worin R² eine Alkylgruppe ist.

6. Verfahren gemäss Anspruch 1, worin Y in den Formeln (III), (IV) und (V) Sauerstoff ist.

7. Verfahren gemäss Anspruch 1, worin Ar in den Formeln (III), (IV) und (V) p-Phenylen oder eine substituierte p-Phenylengruppe ist.

8. Verfahren gemäss Anspruch 1, worin das aromatische Aldehyd durch Verwendung eines Chloritsalzes als Oxidationsmittel oxidiert wird.

9. Verfahren gemäss Anspruch 1, worin mindestens ein Schritt in Gegenwart eines Polymerisationsinhibitors durchgeführt wird.

10. Verfahren zur Herstellung eines aromatischen Aldehyds, dargestellt durch Formel (IV), das die folgenden Schritte umfasst: Synthese eines Sulfonsäureesters, dargestellt durch Formel (II), durch Umsetzung eines (Meth)acrylsäureesters, dargestellt durch Formel (I), mit einem Sulfonyldonor; und Synthese eines aromatischen Aldehyds, dargestellt durch Formel (IV), durch Umsetzung des Sulfonsäureesters, dargestellt durch Formel (II), mit einem aromatischen Aldehyd, dargestellt durch Formel (III): 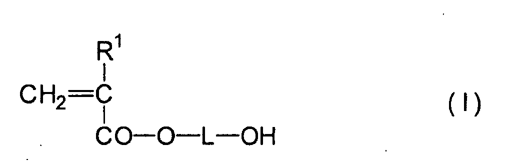 worin L eine divalente Bindungsgruppe mit 2 bis 20 Kohlenstoffatomen ist, ausgewählt aus der Gruppe, bestehend aus einer divalenten, aliphatischen Gruppe, einer divalenten, aromatischen Gruppe und einer Kombination von zwei oder mehreren divalenten, aliphatischen oder aromatischen Gruppen, kombiniert mit -O-, -S-, -CO-, -NH- oder einer Kombination davon; und R¹ ist Wasserstoff oder Methyl; 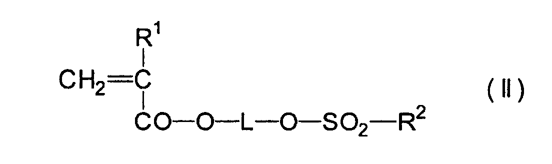 worin L eine divalente Bindungsgruppe mit 2 bis 20 Kohlenstoffatomen ist, ausgewählt aus der Gruppe, bestehend aus einer divalenten, aliphatischen Gruppe, einer divalenten, aromatischen Gruppe und einer Kombination von zwei oder mehreren divalenten, aliphatischen oder aromatischen Gruppen, kombiniert mit -O-, -S-, -CO-, -NH- oder einer Kombination davon; R¹ ist Wasserstoff oder Methyl und R² ist eine aliphatische Gruppe oder eine aromatische Gruppe;
H-Y-Ar-CHO (III)
worin Y Sauerstoff oder Schwefel ist und Ar eine divalente, aromatische Gruppe ist; 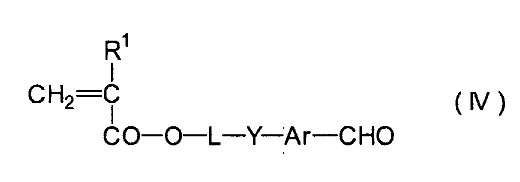 worin L eine divalente Bindungsgruppe mit 2 bis 20 Kohlenstoffatomen ist, ausgewählt aus der Gruppe, bestehend aus einer divalenten, aliphatischen Gruppe, einer divalenten, aromatischen Gruppe und einer Kombination von zwei oder mehreren divalenten, aliphatischen oder aromatischen Gruppen, kombiniert mit -O-, -S-, -CO-, -NH- oder einer Kombination davon; und R¹ ist Wasserstoff oder Methyl; Y ist Sauerstoff oder Schwefel und Ar ist eine divalente, aromatische Gruppe.

## Revendications

1. Procédé pour la préparation d'un acide carboxylique aromatique représenté par la formule (V), qui comprend les étapes : de synthétiser un ester sulfonique représenté par la formule (II) par une réaction d'un ester méth(acrylique) représenté par la formule (I) avec un donneur de sulfonyle ; de synthétiser un aldéhyde aromatique représenté par la formule (IV) par une réaction de l'ester sulfonique représenté par la formule (II) avec un aldéhyde aromatique représenté par la formule (III) ; et de synthétiser l'acide carboxylique aromatique représenté par la formule (V) par une oxydation de l'aldéhyde aromatique représenté par la formule (IV) : 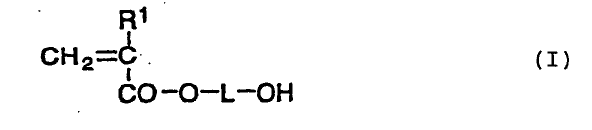 dans laquelle L est un groupe de liaison divalent ayant 2 à 20 atomes de carbone choisi dans le groupe constitué par un groupe aliphatique divalent, un groupe aromatique divalent et une combinaison de deux ou plus de groupes aromatiques ou aliphatiques divalents combinés avec -O-, -S-, -CO-, -NH- ou une combinaison de ceux-ci ; et R¹ est un hydrogène ou un méthyle ; 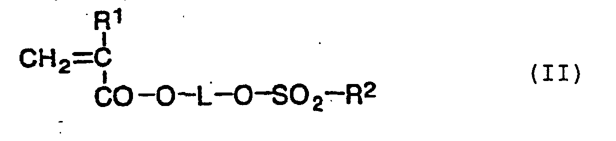 dans laquelle L est un groupe de liaison divalent ayant 2 à 20 atomes de carbone choisi dans le groupe constitué par un groupe aliphatique divalent, un groupe aromatique divalent et une combinaison de deux ou plus de groupes aromatiques ou aliphatiques divalents combinés avec -O-, -S-, -CO-, -NH- ou une combinaison de ceux-ci ; et R¹ est un hydrogène ou un méthyle ; et R² est un groupe aliphatique ou un groupe aromatique ;
H-Y-Ar-CHO (III)
dans laquelle Y est un oxygène ou un soufre ; et Ar est un groupe aromatique divalent ; 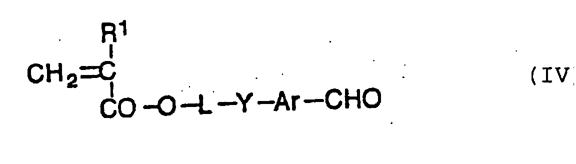 dans laquelle L est un groupe de liaison divalent ayant 2 à 20 atomes de carbone choisi dans le groupe constitué par un groupe aliphatique divalent, un groupe aromatique divalent et une combinaison de deux ou plus de groupes aromatiques ou aliphatiques divalents combinés avec -O-, -S-, -CO-, -NH- ou une combinaison de ceux-ci ; et R¹ est un hydrogène ou un méthyle ; Y est un oxygène ou un soufre ; et Ar est un groupe aromatique divalent ; 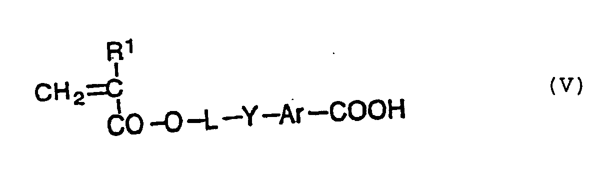 dans laquelle L est un groupe de liaison divalent ayant 2 à 20 atomes de carbone choisi dans le groupe constitué par un groupe aliphatique divalent, un groupe aromatique divalent et une combinaison de deux ou plus de groupes aromatiques ou aliphatiques divalents combinés avec -O-, -S-, -CO-, -NH- ou une combinaison de ceux-ci ; et R¹ est un hydrogène ou un méthyle ; Y est un oxygène ou un soufre ; et Ar est un groupe aromatique divalent.

2. Procédé comme revendiqué dans la revendication 1, dans lequel L dans les formules (I), (II), (IV) et (V) est un groupe de liaison divalent ayant 2 à 20 atomes de carbone choisi dans le groupe constitué par un groupe aliphatique divalent et une combinaison de deux ou plus de groupes aliphatiques divalents combinés avec -O- ou -S-.

3. Procédé comme revendiqué dans la revendication 1, dans lequel L dans les formules (I), (II), (IV) et (V) est un groupe de liaison divalent ayant 2 ou 4 carbones.

4. Procédé comme revendiqué dans la revendication 1, dans lequel le donneur de sulfonyle est un halogénure de sulfonyle représenté par la formule (VI-a) ou un anhydride sulfonique représenté par la formule (VI-b) :
X-SO₂-R₂ (VI-a)
R²-SO₂-O-SO₂-R² (VI-b)
dans lesquelles X est un atome d'halogène; et R² est un groupe aliphatique ou un groupe aromatique.

5. Procédé comme revendiqué dans la revendication 4, dans lequel R² est un groupe alkyle.

6. Procédé comme revendiqué dans la revendication 1, dans lequel Y dans les formules (III), (IV) et (V) est un oxygène.

7. Procédé comme revendiqué dans la revendication 1, dans lequel Ar dans les formules (III), (IV) et (V) est un p-phénylène ou un groupe p-phénylène substitué.

8. Procédé comme revendiqué dans la revendication 1, dans lequel l'aldéhyde aromatique est oxydé en utilisant un sel de chlorate en tant qu'agent oxydant.

9. Procédé comme revendiqué dans la revendication 1, dans lequel au moins une étape est conduite en présence d'un inhibiteur de polymérisation.

10. Procédé pour la préparation d'un aldéhyde aromatique représenté par la formule (IV), qui comprend les étapes : de synthétiser un ester sulfonique représenté par la formule (II) par une réaction d'un ester méth(acrylique) représenté par la formule (I) avec un donneur de sulfonyle ; et de synthétiser un aldéhyde aromatique représenté par la formule (IV) par une réaction de l'ester sulfonique représenté par la formule (II) avec un aldéhyde aromatique représenté par la formule (III) : 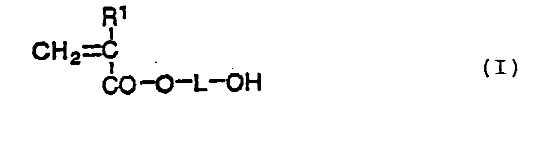 dans laquelle L est un groupe de liaison divalent ayant 2 à 20 atomes de carbone choisi dans le groupe constitué par un groupe aliphatique divalent, un groupe aromatique divalent et une combinaison de deux ou plus de groupes aromatiques ou aliphatiques divalents combinés avec -O-, -S-, -CO-, -NH- ou une combinaison de ceux-ci ; et R¹ est un hydrogène ou un méthyle ; 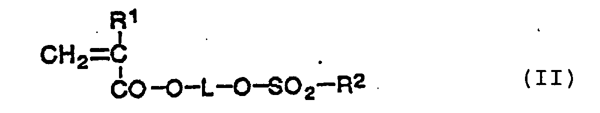 dans laquelle L est un groupe de liaison divalent ayant 2 à 20 atomes de carbone choisi dans le groupe constitué par un groupe aliphatique divalent, un groupe aromatique divalent et une combinaison de deux ou plus de groupes aromatiques ou aliphatiques divalents combinés avec -O-, -S-, -CO-, -NH- ou une combinaison de ceux-ci ; et R¹ est un hydrogène ou un méthyle ; et R² est un groupe aliphatique ou un groupe aromatique ;
H-Y-Ar-CHO (III)
dans laquelle Y est un oxygène où un soufre ; et Ar est un groupe aromatique divalent ; 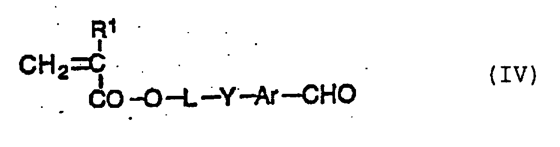 dans laquelle L est un groupe de liaison divalent ayant 2 à 20 atomes de carbone choisi dans le groupe constitué par un groupe aliphatique divalent, un groupe aromatique divalent et une combinaison de deux ou plus de groupes aromatiques ou aliphatiques divalents combinés avec -O-, -S-, -CO-,- -NH- ou une combinaison de ceux-ci ; et R¹ est un hydrogène ou un méthyle ; Y est un oxygène ou un soufre ; et Ar est un groupe aromatique divalent.
